# EUROPÄISCHE PATENTSCHRIFT

(11) **EP 3 954 817 B1**
(45) Veröffentlichungstag und Bekanntmachung des Hinweises auf die Patenterteilung: **23.10.2024**
(21) Anmeldenummer: 20020363.6
(22) Anmeldetag: 11.08.2020
(51) Int. Cl.: D04B 1/24, D04B 1/26, A61F 13/08

(54) **BEINBEKLEIDUNGSSTÜCK**
PIECE OF CLOTHING FOR THE LEGS
VÊTEMENT POUR JAMBES

(43) Veröffentlichungstag der Anmeldung: 16.02.2022
(73) Patentinhaber: medi GmbH & Co. KG, 95448 Bayreuth (DE)
(72) Erfinder: Rockstroh, Martin, 95473 Creußen (DE)

(56) Entgegenhaltungen:
- CA-A- 784 903
- DE-A1- 102018 004 065
- DE-U- 1 772 612
- GB-A- 2 316 418
- JP-A- H11 350 203
- US-A1- 2018 353 345

## Beschreibung

Die vorliegende Erfindung bezieht sich auf ein Beinbekleidungsstück nach dem Oberbegriff des Anspruch 1.

Derartige Beinbekleidungsstücke werden insbesondere in Form von Füßlingen, Socken, Strümpfen, insbesondere als Waden- oder Oberschenkelstümpfe, oder beispielsweise als Strumpfhosen ausgebildet. Für den medizinischen Einsatz und/ oder für die Anwendung bei sportlichen Aktivitäten weisen diese oftmals einen oder mehrere kompressive Bereiche auf, welche dazu dienen gezielt Druck auf den Körper eines Patienten auszuüben. Der auf den Körper eines Patienten ausgeübte Druck wird als Kompression bezeichnet. Ziel bei kompressiven Beinbekleidungsstücken beispielsweise, insbesondere für den medizinischen Einsatz, ist es unter anderem ein geschädigtes Venen- und/ oder Lymphsystem eines Patienten zu entlasten. Durch den zugeführten Druck wird eine zunehmende Schwellung der Gliedmaßen vermieden, der Abtransport von venösem Blut und Lymphe verbessert und die Blutzufuhr gesteigert. Bei Einsatz von kompressiven Beinbekleidungsstücken in dem Sportbereich bewirken diese eine Leistungssteigerung bzw. eine verbesserte Regeneration.

Zur Ausbildung dieser, vorzugsweise kompressiven, Beinbekleidungsstücke, werden diese mittels einer Rundstrickmaschine rundgestrickt oder mittels einer Flachstrickmaschine, flachgestrickt und im Anschluss mittels einer Längsnaht zu einem Schlauchgestrick zusammengenäht. Das Grundgestrick ist hierbei aus wenigstens einem maschenbildenden Grundstrickfaden über mehrere Maschenreihen gestrickt. Zur Bildung kompressiver Gestrickteile wird in die gestrickten Maschen vorzugsweise ein elastischer Schussfaden in jeder oder in jede X-ten, vorzugsweise in jeder zweiten, Reihe eingelegt. Zur Herstellung, insbesondere zur Messung und Gütesicherung, von kompressiven Arm- oder Beinstrümpfen für die medizinische Anwendung, existiert die RAL-GZ 387 der Gütezeichengemeinschaft. Aus den Prüfbestimmungen der RAL kann entnommen werden, wie der Druck eines Kompressionsstrumpfes auf ein Bein zu bestimmen ist. Als Messmittel, insbesondere Kompressionsprüfgerät, wird die Prüfung am HOSY Messgerät (Institut Hohenstein) vorgeschlagen. Die Prüfung erfolgt u.a. anhand der Messung der Spannkraft in den mehreren Messpunkten, welche abhängig von der jeweiligen Dehnbarkeit des Gestricks variiert. Aus der Spannkraft in Kombination mit weiteren das Gestrick betreffenden Parametern wird die Kompression errechnet. D.h. je mehr Dehnbarkeit ein Gestrick in Gestrickquerrichtung aufweist, desto geringer ist die durch das Gestrick am Patienten ausgeübte Kompression. Andererseits je geringer die Dehnbarkeit des Gestrickteils ist, desto höher ist die am Patienten ausgeübte Kompression.

Aus dem Stand der Technik sind eine Vielzahl von, vorzugsweise flachgestrickten, Beinbekleidungsstücken, insbesondere in Form von Beintrümpfen, bekannt, welche mittels einer Längsnaht zu einem Schlauchgestrick zusammengenäht sind.

Ein derartiges Beinbekleidungsstück, insbesondere in Form eines kompressiven Strumpfes, wird beispielsweise in der DE 1 772 612 U offenbart.

Dieser bekannte Strumpf, auch als gummielastische Bandage bezeichnet, ist durch eine besondere Naht gekennzeichnet, welche im getragenen Zustand nicht mehr aufträgt, also äußerst flach ausgebildet ist und somit keine auftragende Wulst bildet. Hierzu werden die Kanten stumpf aneinandergelegt und mittels einer Spezial-Flachnaht-Maschine miteinander vernäht. Bisher wurden die Verbindungsnähte durch Überlappung der Kanten zusammengenäht, was zu einer breiten Wulst an der Verbindungsstelle führte. Die dadurch erhöhte Kompression führte beim Träger des Strumpfes zu Schmerzen im Bereich der Naht. Ferner ist der Strumpf hierbei derart ausgebildet, dass die Naht des Strumpfes entlang dessen Rückseite, insbesondere mittig an der Rückseite, verläuft.

Diese bekannte Anordnung der Naht bei einem flachgestrickten, insbesondere kompressiven, Beinbekleidungsstück ist beispielsweise auch aus der CA 784903 A bekannt.

Die CA 784903 A offenbart ebenfalls einen kompressiven Beinstrumpf, insbesondere in Form eine Oberschenkelstrumpfes. Dieser, insbesondere das dort offenbarte erfindungsgemäße Gestrick, wird hierbei wahlweise mittels einer Rundstrickmaschine oder mit ein Flachstrickmaschine hergestellt. Bei Ausbildung des Strumpfes als Rundgestrick, wird der Strumpf vorzugsweise nahtlos gestrickt. Bei Fertigung des Strumpfes durch ein Flachgestrick, wird dieses mittels einer Längsnaht zu einem Schlauchgestrick, insbesondere zu einem Strumpf, zusammengenäht. Auch hier ist das Flachgestrick derart ausgebildet, dass die Naht des Strumpfes entlang dessen Rückseite, insbesondere mittig an der Rückseite, verläuft. Konkret beginnt die Naht an dem unteren Strumpfende, nämlich der Unterseite der Zehenteils des Strumpfes, verläuft mittig entlang der Fußsohle und mittig durch einen Fersenbereich des Strumpfes. Anschließend ist die Naht mittig an der Rückseite des Strumpfes, nämlich in einem Wadenbereich und der Kniekehle, angeordnet und verläuft bis zu einem oberen Strumpfende, welches durch ein Haftband gebildet ist.

Die GB 2 316 418 A offenbart ferner eine gestrickte Socke, welche durch einen flachgestrickten und länglichen Rohling gebildet ist. Zur Ausbildung der schlauchförmigen Socke wir der Rohling in einem mittleren Bereich, nämlich in einem Zehenbereich, in der Länge gefalten. Die übereinander liegenden Flächen des Rohlings werden anschließend mittels zweier Nähte zusammengenäht. Die fertige Socke weist dadurch zwei seitlich angeordnete medial und lateral am Fuß verlaufende Längsnähte auf, die sich von dem Zehenbereich bis zu einem oberen offenen Ende der Socke erstrecken.

Auch die JP H11 350203 offenbart eine derartige Socke, welche durch ein flaches und längliches Gestrickteil ausgebildet ist, das in einem mittleren Bereich in der Länge gefalten wird, um eine schlauchförmige Socke auszubilden. Das gefaltene Gestrickteil wird ebenfalls an beiden Seiten durch zwei Nähte geschlossen.

Als nachteilig hierbei erweist sich, dass zur Ausbildung der Socken, wie in den beiden zuvor genannten Druckschriften beschrieben, mehrere, zumindest zwei beabstandete, Nähte notwendig sind, was einen schlechteren Tragekomfort zur Folge hat. Nähte in einem Kleidungsstück, insbesondere in einem Beinbekleidungsstück, sollten möglichst vermieden werden.

Als nachteilig der eingangs beschriebenen und aus dem Stand der Technik bekannten und allgemein üblichen Anordnung der Längsnaht bei einem, vorzugsweise kompressiven und flachgestrickten, Beinbekleidungsstück hat sich ebenfalls der schlechte Tragekomfort erwiesen.

Die Ausbildung des Flachgestricks derart, dass die Naht in einem getragenen Zustand im Bereich der Fußsohle angeordnet ist, insbesondere in einem Fersenbereich, führt dazu, dass der Träger des Strumpfes bei jedem Kontakt mit dem Boden auf die Naht tritt. Hierbei übt das ganze Körpergewicht des Trägers einen Druck auf die Naht aus, was beim Träger zu Schmerzen im Bereich der Naht führt, insbesondere dann, wenn bspw. zur Verbesserung der Nahthaltbarkeit ein zusätzliches Nahtband verwendet wird, welches auf die beiden Gestrickkanten des Flachgestricks genäht wird. Auch die Anordnung der Naht im Wadenbereich und der dortigen erhöhten Kompression, insbesondere verursacht durch die Aktivität der Wadenmuskulaturpumpe, kann zu Schmerzen im Bereich der Naht führen. Schließlich kommt es auch auf Grund einer Faltenbildung, bedingt durch die Naht, im Bereich einer Kniekehle zu einem geringeren Tragekomfort bei Ausbildung des Strumpfes als Oberschenkelstrumpf.

Ein weiterer Nachteil der aus dem Stand der Technik bekannten Beinbekleidungsstücke ist, dass die Strümpfe für Diabetiker oder Allergiker, insbesondere Personen mit empfindlichen Füßen, ungeeignet sind. Wie bereits zuvor erwähnt, kommt es stellenweise durch die Anordnung der Naht im Bereich der Fußsohle zu einem erhöhten Druck auf die Fußsohle. Bei Patienten ohne Schmerzempfinden und chronischen Wunden im Bereich des Fußes, insbesondere im Bereich der Fußsohle, kann dies zu einer weiteren Reizung der Wunde führen. Aufgrund schlechter Wundheilung bei Patienten, die an Diabetes leiden, ist dies unbedingt zu vermeiden.

Schließlich erweist sich auch als nachteilig, dass durch die Anordnung der Naht in Zonen mit erhöhter Beanspruchung, nämlich der Fußsohle, der Ferse, im Bereich der Wadenmuskulaturpumpe oder im Bereich der Kniekehle, die Haltbarkeit des Strumpfes stark beeinträchtigt wird. In den genannten Zonen kommt es selbst bei gewöhnlicher Beanspruchung zu erhöhten Druckbelastungen, Reibungskräften, als auch Zugkräften auf die Naht bedingt durch die Dehnung des Gestricks, insbesondere verursacht durch die Bewegung zweier über ein Gelenk, bspw. dem Sprunggelenk, miteinander verbundenen Körperteilen.

Der vorliegenden Erfindung liegt daher die Aufgabe zugrunde ein Beinbekleidungsstück zu schaffen, welches die Nachteile aus dem Stand der Technik vermeidet, insbesondere den Tragekomfort eines flachgestrickten, vorzugweise kompressiven, und mittels einer Längsnaht geschossenen Beinbekleidungsstücks deutlich verbessert.

Gemäß einem Ausführungsbeispiel weist das Beinbekleidungsstück ein zumindest ein Fußteil ausbildendes flachgestricktes Grundgestrickteil mit zumindest einer ersten in das Grundgestrickteil eingestrickten Funktionszone auf, welche zur Aufnahme einer Ferse eines Fußes ausgebildet ist, wobei das flachgestrickte Grundgestrickteil mittels einer Längsnaht zu einem Schlauchgestrick zusammengenäht ist und wobei die zumindest eine erste Funktionszone derart in das Grundgestrickteil eingestrickt ist, dass die das Grundgestrickteil verbindende Längsnaht im getragenen Zustand des Beinbekleidungsstücks zumindest in einem Fersenbereich außerhalb der Fußsohle angeordnet ist.

Gemäß einem zweiten Ausführungsbeispiel erstreckt sich die Längsnaht des Grundgestrickteils im getragenen Zustand des Beinbekleidungsstücks vorzugsweise zumindest teilweise medial oder lateral an der Seite des Fußes und/ oder über die Fußoberseite. Besonders bevorzugt erstreckt sich die Längsnaht im getragenen Zustand des Beinbekleidungsstücks vorzugsweise über die gesamte Fußlänge medial oder lateral an der Seite des Fußes und/ oder über die Fußoberseite. Dadurch ist die Fußsohle frei von jeglichen Nähten, was zu einem deutlich besseren Tragekomfort führt.

Gemäß einem dritten Ausführungsbeispiel des Beinbekleidungsstücks bildet das flachgestrickte Grundgestrickteil zusätzlich ein Zehenteil aus, wobei das Zehenteil mittels einer Quernaht zu einer geschlossenen Fußspitze zusammengenäht ist. Bei Ausbildung des Fußteiles derart, dass die Längsnaht des Fußteiles im getragenen Zustand des Beinbekleidungsstücks medial oder lateral an der Seite des Fußes verläuft, ist bevorzugt die Längsnaht des Fußteils und die Quernaht des Zehenteils als eine durchgängige Naht ausgebildet. Hierdurch kann durch einen Fertigungsschritt, nämlich einer einzigen Naht, das gesamte Beinbekleidungsstück, insbesondere das flachgestrickte Grundgestrickteil, geschlossen werden. Eine zweite Naht ist nicht erforderlich.

Gemäß einem vierten Ausführungsbeispiel des Beinbekleidungsstücks bildet das flachgestrickte Grundgestrickteil zusätzlich ein Unterschenkelteil aus, wobei sich die Längsnaht im getragenen Zustand des Beinbekleidungsstücks medial oder lateral an der Seite des Unterschenkels und/ oder über die Vorderseite, insbesondere entlang dem Schienbeinknochen, erstreckt. Bevorzugt erstreckt sich die Naht an jener Seite, also medial, lateral oder an der Vorderseite des Beines, in der Art und Weise, wie sie sich auch im anschließenden Fußteil erstreckt. D.h. erstreckt sich die Naht im Fußteil bspw. medial an der Innenseite des Fußes, dann erstreckt sich die Naht im Unterschenkelteil bevorzugt ebenfalls medial, also an der Innenseite des Beines. Entsprechendes gilt für die laterale Platzierung oder Anordnung an der Naht an der Oberseite des Fußes. Alternativ ist es natürlich auch möglich, dass das Beinbekleidungsstück derart ausgebildet ist, dass die Anordnung der Naht im getragenen Zustand des Beinbekleidungsstücks sich zwischen Fußteil und Unterschenkelteil unterscheidet. Es sind sämtliche Kombinationen denkbar, besonderes bevorzugt bspw.: Naht im Fußteil medial und im Unterschenkelteil an der Vorderseite, Naht im Fußteil lateral und im Unterschenkelteil an der Vorderseite, Naht im Fußteil an der Fußoberseite und im Unterschenkelteil medial, Naht im Fußteil an der Fußoberseite und im Unterschenkelteil lateral. Die Auflistung ist nicht abschließend.

Gemäß einem fünften Ausführungsbeispiel des Beinbekleidungsstücks bildet das flachgestrickte Grundgestrickteil zuätzlich zu dem Unterschenkelteil ein Oberschenkelteil aus, wobei sich die Längsnaht im getragenen Zustand des Beinbekleidungsstücks medial oder lateral an der Seite des Oberschenkels und/ oder über die Vorderseite des Oberschenkels erstreckt. Auch hier erstreckt sich die Naht bevorzugt an jener Seite, also medial, lateral oder an der Vorderseite des Beines, in der Art und Weise, wie sie sich auch im anschließenden Unterschenkelteil und Fußteil erstreckt. Aber auch hier kann sich die Erstreckung der Naht zwischen Fußteil, Unterschenkelteil und/ oder Oberschenkelteil unterscheiden. Bspw. erstreckt sich die Naht im Fußteil an der Fußoberseite, um einen Knöchelbereich frei von einer Naht zu halten und erstreckt sich dann medial oder lateral im Bereich des Unterschenkels und Oberschenkels, so dass ein Kniebereich und eine Kniekehle ebenfalls frei von einer Naht ist. Denkbar ist aber auch, dass sich die Längsnaht in allen drei Bereichen, also im Fußteil, Unterschenkelteil und Oberschenkelteil im getragenen Zustand des Beinbekleidungsstücks an unterschiedlichen Seiten erstreckt, um einen maximalen Tragekomfort zu erhalten.

Gemäß einem sechsten Ausführungsbeispiel des Beinbekleidungsstücks bildet das flachgestrickte Grundgestrickteil zusätzlich zu dem Oberschenkelteil ein Leibteil aus, wobei sich die Längsnaht im getragenen Zustand des Beinbekleidungsstücks an der Seite des Beckens und/ oder über die Vorderseite des Beckens erstreckt. Auch hier kann die Erstreckung der Längsnaht im Fußteil, Unterschenkelteil, Oberschenkelteil und/ oder Leibteil in der gleichen Art und Weise erfolgten, also bspw. durchgängig medial an der Seite des Beines, oder sich unterscheiden.

Gemäß einem weiteren Ausführungsbeispiel weist das flachgestrickte Grundgestrickteil zumindest eine zweite in das Grundgestrickteil eingestrickten Funktionszone auf, welche bevorzugt gegenüber dem flachgestrickten Grundgestrickteil eine höhere Elastizität in Längs- und/ oder Querrichtung aufweist. Die zweite Funktionszone kann bspw. als Entlastungszone im Knöchelbereich oder im Bereich des Spanns dienen, insbesondere um dort eine Faltenbildung zu vermeiden.

Gemäß einem weiteren Ausführungsbeispiel sind die zwischen einem Gestrickanfang und einem Gestrickende verlaufenden und gegenüberliegenden Gestrickkanten des flachgestrickten Grundgestrickteils symmetrisch, insbesondere spiegelsymmetrisch, zueinander ausgebildet. Besonderes bevorzugt sind die zwischen einem Gestrickanfang und einem Gestrickende verlaufenden und gegenüberliegenden Gestrickkanten des flachgestrickte Grundgestrickteils asymmetrisch zueinander ausgebildet. Dies ermöglicht eine Führung der Längsnaht entlang dem Bein eines Trägers, insbesondere vorbei an empfindlichen Stellen am Bein eines Trägers, wie bspw. dem Knöchel, der Kniekehle oder vorbei an anderen lokalen empfindlichen Stellen, wie zum Beispiel Wunden. Besonders bevorzugt wird der Verlauf der Gestrickkanten und somit der Verlauf der Längsnaht entlang des Fußteiles, des Unterschenkelteils, des Oberschenkelteils und/ oder des Leibteils individuell, also personenabhängig, festgelegt. Insbesondere bei einer Maßversorgung, bei der der Patient ein maßgefertigtes Beinbekleidungsstück erhält und das Grundgestrickteil somit nach Maß gestrickt wird, ist es hierdurch möglich die Position der Längsnaht am Bein eines Trägers individuell zu bestimmen und festzulegen.

Gemäß einem weiteren Ausführungsbeispiel ist das flachgestrickte Grundgestrickteil durch einen oder mehrere maschenbildende Grundstrickfäden sowie zumindest einen eingelegten Schussfaden gebildet. Besonders bevorzugt weist es hierbei in Gestricklängsrichtung einen graduellen Kompressionsverlauf auf. Der graduelle Druckverlauf wird bevorzugt durch die Art und Weise, insbesondere über die Anzahl von aufeinanderfolgenden Maschenreihen, in denen der Schussfaden hinterlegt wird, bestimmt. Die Kompressionsstärke nimmt vorzugsweise kontinuierlich, in Gestrickslängsrichtung ab oder zu. Die durch das flachgestrickte Grundgestrickteil erzeugten kompressiven Drücke betragen hierbei in einem Fesselbereich des Beinbekleidungsstücks bevorzugt zwischen 10 und 60 mmHg und in einem Wadenbereich zwischen 5 und 50 mmHg.

Gemäß einem weiteren Ausführungsbeispiel ist das Beinbekleidungsstück einteilig oder mehrteilig ausgebildet. D.h. die mehreren Teile, nämlich das Fußteil, das Unterschenkelteil, das Oberschenkelteil und/ oder das Leibteil sind durch ein einziges durchgängig flachgestricktes Grundgestrickteil gebildet, oder durch zwei oder mehrere getrennt voneinander ausgebildete Grundgestrickteile, welche jeweils durch eine Längsnaht zu einem Schlauchgestrick zusammengenäht sind. Die mehrteilige Ausbildung ermöglicht eine leichtere Anziehbarkeit.

Das Beinbekleidungsstück nach den vorherigen Ausführungsbeispielen ist bevorzugt als Füßling, Socke, Strumpf, insbesondere als Waden- oder Oberschenkelstumpf, als Strumpfhose, Fußbandage, oder als Strickteil einer Fußorthese ausgebildet. Besonders bevorzugt ist es als ein kompressives Beinbekleidungsstück ausgebildet, wobei die Kompressionsstärke in Gestricklängsrichtung, insbesondere von der Fessel in Richtung oberes Gestrickende, ab oder zunimmt.

Das vorliegende Beinbekleidungsstück zeichnet sich durch eine Reihe erheblicher Vorteile aus.

Durch die Ausbildung des Beinbekleidungsstücks derart, dass die das Grundgestrickteil des Beinbekleidungsstücks verbindende Längsnaht im getragenen Zustand des Beinbekleidungsstücks zumindest in einem Fersenbereich außerhalb der Fußsohle angeordnet ist, wird ein deutlich besserer Tragekomfort erreicht. Zwischen dem Fuß des Trägers und einer Auftrittsfläche ist keine störende Nahtverbindung, welche beim Träger zu Schmerzen im Bereich der Naht führen kann, angeordnet. Die Fußsohle des Beinbekleidungsstücks ist somit vorzugsweise frei von jeglichen Nahtverbindungen. Diese besteht somit vorzugsweise lediglich aus dem gleichmäßig gestrickten Grundgestrickteil. Es sind jedenfalls keine reizauslösenden Elemente des Grundgestrickteils in der Fußsohle des Beinbekleidungsstücks enthalten.

Ferner ist bei Ausbildung des Beinbekleidungsstücks bspw. als Waden- oder Oberschenkelstrumpf, durch die Anordnung der Längsnaht im getragenen Zustand des Beinbekleidungsstücks medial oder lateral an der Seite des Unterschenkels oder an der Vorderseite, also im Bereich des Schienbeinknochens, die Naht nicht im Wadenbereich des Beines eines Trägers angeordnet. Der hintere Wadenbereich ist somit ebenfalls frei von jeglichen Nahtverbindungen. Die Aktivität der Wadenmuskulaturpumpe führt somit nicht zu Schmerzen im Bereich des Unterschenkels, auf Grund von störenden bzw. reizauslösenden Elementen im Wadenbereich des Grundgestrickteils.

Schließlich bringt die Anordnung der Längsnaht im getragenen Zustand des Beinbekleidungsstücks medial oder lateral an der Seite oder an der Vorderseite des Unterschenkels und des Oberschenkels den Vorteil mit sich, dass keine störende Nahtverbindung im Bereich der Kniekehle angeordnet ist. Dies führt ebenfalls, auf Grund der geringeren Faltenbildung, zu einem deutlich besseren Tragekomfort.

Ein weiterer Vorteil der Erfindung ist, dass das erfindungsgemäße Beinbekleidungsstück insbesondere auch für empfindliche Füße geeignet ist, also insbesondere für Diabetiker oder Allergiker. Dadurch, dass die Fußsohle vorzugsweise frei von jeglichen Nähten ist, erfolgt keine Reizung der Fußsohle, insbesondere von Wunden im Bereich der Fußsohle bei Patienten ohne Schmerzempfinden und chronischen Wunden im Bereich des Fußes.

Besonders vorteilhaft ist auch, dass durch das erfindungsgemäße Beinbekleidungsstücke die Längsnaht des Grundgestrickteils außerhalb jener Bereiche des Beinbekleidungsstücks angeordnet ist, die durch den Gebrauch einer erhöhten Beanspruchung ausgesetzt sind, insbesondere die Fußsohle und Ferse. Dies führt schließlich dazu, dass die Haltbarkeit des Beinbekleidungsstücks deutlich verbessert wird.

Nachfolgend wird die Erfindung anhand mehrerer Ausführungsbeispiele und in Verbindung mit den beigefügten Zeichnungen erläutert.

Dabei zeigt:
Figur 1 ein erstes Ausführungsbeispiel des erfindungsgemäßen Beinbekleidungsstücks, welches ein flachgestricktes Grundgestrickteil aufweist und als Socke mit offener Spitze ausgebildet ist,
Figur 2 ein zweites Ausführungsbeispiel des erfindungsgemäßen Beinbekleidungsstücks, welches ein flachgestricktes Grundgestrickteil aufweist und als Strumpf, insbesondere als Wadenstrumpf, ausgebildet ist,
Figur 3 ein drittes Ausführungsbeispiel des erfindungsgemäßen Beinbekleidungsstücks, welches ebenfalls wie in Figur 2 gezeigt als Strumpf, insbesondere als Wadenstrumpf, ausgebildet ist,
Figur 4 ein viertes Ausführungsbeispiel des erfindungsgemäßen Beinbekleidungsstücks, welches als Oberschenkelstrumpf ausgebildet ist,
Figur 5 ein fünftes Ausführungsbeispiel des erfindungsgemäßen Beinbekleidungsstücks, welches als Strumpfhose ausgebildet ist,
Figur 6 eine schematische Darstellung des Strickablaufs des flachgestrickten Grundgestrickteils, wie es zur Bildung des Beinbekleidungsstücks, wie es in Figur 1 zu sehen ist, verwendet wird,
Figur 7 eine schematische Darstellung des Strickablaufs es flachgestrickten Grundgestrickteils, insbesondere einen Ausschnitt daraus, wie es in Figur 4 zu sehen ist;

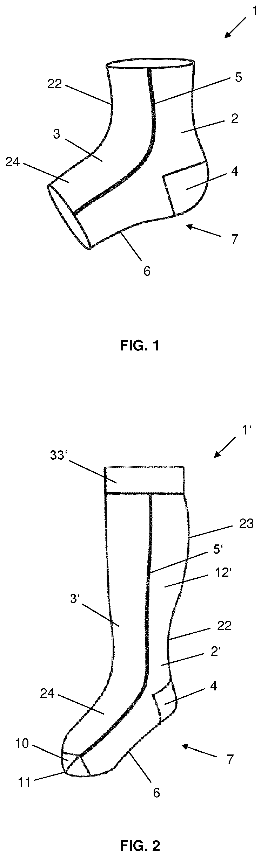

In Figur 1 ist ein erstes Ausführungsbeispiel des Beinbekleidungsstücks 1 in Form einer vorzugsweise kompressiven und vorzugsweise medizinischen Socke mit einer offenen Spitze gezeigt. Das Beinbekleidungsstück 1 besteht aus einem Fußteil 2, welches aus einem über mehreren Maschenreihen flachgestrickten Grundgestrickteil 3 gefertigt ist, das wiederum mittels einer Längsnaht 5 zu einem Schlauchgestrick zusammengenäht ist. In dem Grundgestrickteil 3 ist eine erste Funktionszone 4 eingestrickt, welche zur Aufnahme einer Ferse eines Fußes ausgebildet ist. Die erste Funktionszone 4 ist hierbei derart in das Grundgestrickteil 3 eingestrickt, dass die das Grundgestrickteil verbindende Längsnaht 5 im getragenen Zustand des Beinbekleidungsstücks 1 zumindest in einem Fersenbereich außerhalb der Fußsohle 6 angeordnet ist. In dem gezeigten Ausführungsbeispiel verläuft die Naht 5 medial an der Innenseite 7 des Fußes eines Trägers des Beinbekleidungsstücks 1, also an der Innenseite im Mittelfußbereich 24, dem Fersenbereich als auch dem Fesselbereich 22. Die Fußsohle 6 weist somit keine für den Träger des Beinbekleidungsstücks 1 störende Nahtverbindung auf.

Das Grundgestrickteil 3 selbst ist gemäß diesem und der folgenden Ausführungsbeispiele als ein einlagiges oder mehrlagiges Flachgestrick ausgebildet. Vorzugsweise ist das Grundgestrickteil 3 durch eine Rechts-Rechtsbindung, bestehend aus einem RR-Strickfaden (Rechts-Rechts-Strickfaden) und einem eingelegten Schussfaden, oder durch eine Halbschlauchbindung bestehend aus einem RR-Strickfaden, einem RL-Strickfaden (Rechts-Links-Strickfaden) und dem eingelegten Schussfaden gebildet.

Die Längsnaht 5 wiederum weist zur verbesserten Nahthaltbarkeit bzw. Nahtfestigkeit gemäß diesem und der folgenden Ausführungsbeispiele vorzugweise ein Nahtband auf. Hierbei handelt es sich um ein bandförmiges Textilelement, welches gemeinsam mit den beiden Gestrickkanten des Flachgestricks vernäht wird. Alternativ kann die Längsnaht 5 auch nur als überlappende oder Stoß-an-Stoß Verbindung ausgebildet sein.

Gemäß einem bevorzugten Ausführungsbeispiel handelt es sich bei dem Beinbekleidungsstück 1 um eine kompressive Socke, deren erzeugte kompressiven Drücke in einem Fesselbereich 22 bevorzugt zwischen 10 und 60 mmHg, und in einem Mittelfußbereich 24 bevorzugt zwischen 10 und 50 mmHg betragen. Das auf einer Fachstrickmaschine flachgestrickte Grundgestrickteil 3 weist hierzu einen oder mehrere in mehrere Grundstrickmaschenreihen eingelegte Schussfäden auf. Die kompressiven Werte werden mittels der eingangs vorgestellten Messmethodik und Messgerät, insbesondere mittels der Prüfung am HOSY Messgerät (Institut Hohenstein), gemessen.

Figur 2 zeigt ein zweites Ausführungsbeispiel des flachgestrickten Beinbekleidungsstücks 1', welches als Strumpf, insbesondere als Wadenstrumpf, ausgebildet ist. Dieser besteht aus einem ein Fußteil 2' sowie ein Unterschenkelteil 12' ausbildendes, vorzugsweise kompressives, Grundgestrickteil 3'. Zudem weist das Beinbekleidungsstück 1', vorzugsweise das Grundgestrickteil 3', ein Zehenteil 10 auf, wobei das Zehenteil 10 mittels einer Quernaht 11 zu einer geschlossenen Fußspitze zusammengenäht ist. Die gebildete Fußspitze kann somit an das Grundgestrickteil 3' angenäht, oder einteilig mit diesem gestrickt sein. Am gegenüberliegenden Ende des Strumpfes 1' ist an dem Grundgestrickteil 3' ein Haftabschnitt 33' in Form eines, vorzugsweise zweilagigen, Bundes oder eines Haftbandes angestrickt oder angenäht. Dieser dient dazu, dass der Strumpf am Bein des Trägers nicht verrutsch.

Die das flachgestrickte Grundgestrickteil 3' verbindende Längsnaht 5' verläuft gemäß diesem Ausführungsbeispiel, durch die spezielle Anordnung der ersten Funktionszone 4, nämlich der Ferse, in dem Grundgestrickteil 3' und durch die Ausbildung des Grundgestrickteils 3' selbst, sowohl im Mittelfußbereich 24, dem Fesselbereich 22, als auch dem Wadenbereich 23 medial an der Innenseite 7 des Fußes und Beins eines Trägers des Beinbekleidungsstücks 1'. Die der Fußsohle 6 eines Trägers zugewandte Seite des Beinbekleidungsstücks 1 ist somit frei von einer störenden Nahtverbindung. Aber auch der Wadenbereich 23 weist keine störende Nahtverbindung auf.

Bevorzugt ist der gezeigte Wadenstrumpf als kompressives Beinbekleidungsstück 1' ausgebildet. Hierbei betragen die kompressiven Drücke in dem Fesselbereich 22 bevorzugt zwischen 10 und 60 mmHg, und in dem Wadenbereich zwischen 5 und 50 mmHg. Die Kompressionsstärke nimmt hierbei vom unteren Ende in Richtung der Wadenmuskulatur bevorzugt ab. Alternativ ist auch ein ansteigender Druckverlauf möglich.

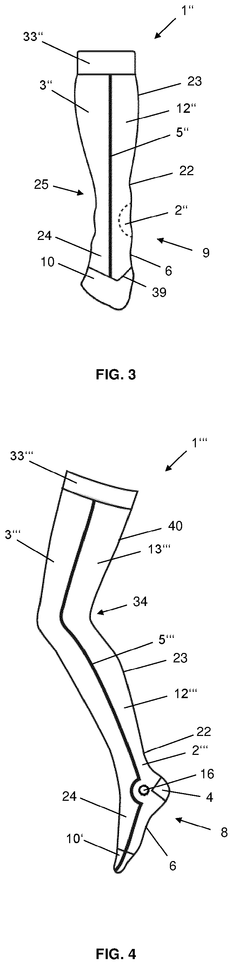

In Figur 3 ist ein drittes Ausführungsbeispiel des Beinbekleidungsstücks 1" gezeigt, welches ebenfalls, wie in Figur 2 gezeigt, als Strumpf, insbesondere als Wadenstrumpf, ausgebildet ist. Der Strumpf 1" weist ebenfalls, neben einem Fußteil 2", ein Unterschenkelteil 12" sowie ein Zehenteil 10 auf. Das Zehenteil 10 ist gemäß diesem Ausführungsbeispiel vorzugsweise an das Grundgestrickteil 3" mittels einer einer Nahtverbindung 39 angenäht. Auch der Haftabschnitt 33" ist vorgesehen. Der Wadenstrumpf unterscheidet sich hierbei jedoch insbesondere dadurch, dass die Funktionszone 4 aus Figur 2, nämlich die vorzugsweise eingestrickte Ferse, derart in das Grundgestrickteil 3" bzw. in das Fußteil 2" eingearbeitet ist, dass sich die Längsnaht 5" im getragenen Zustand des Beinbekleidungsstücks 1" in einem Mittelfußbereich 24 über die Fußoberseite 9 und in einem Fesselbereich 22 sowie Wadenbereich 23 sich über die Vorderseite 25 des Beinbekleidungsstücks 1", insbesondere entlang dem Schienbeinknochen eines Trägers, erstreckt. Die Längsnaht 5" trifft auf der Fußoberseite 9 auf die Nahtverbindung 39. Dadurch ist es möglich, dass die Naht 5" sowie die Naht 39 als eine einzige, insbesondere durchgängige Naht ausgebildet werden kann. Die Fußsohle 6 ist somit in einem hinteren Fersenbereich frei von Nähten, was dazu führt, dass es zu einem deutlich besseren Tragekomfort kommt.

Das Zehenteil 10, insbesondere die gebildete Fußspitze, kann neben der gezeigten geschlossenen Variante auch andere Formen aufweisen, bspw. eine offene Spitze oder mehrere schlauchförmige Gestrickteile für jede einzelne Zehe. Hierbei weisen die offenen oder geschlossenen Fußspitzen vorzugsweise unterschiedliche Druckprofile auf, welche wahlweise mit den Druckprofilen des Fuß- und Unterschenkelteils 2", 12", vorzugsweise nach den mehreren Kompressionsklassen 1 bis 4 nach RAL-GZ 387, kombiniert werden.

In Figur 4 ist ein Beinbekleidungsstücks 1‴ gezeigt, welches als Oberschenkelstrumpf ausgebildet ist. Der Oberschenkelstrumpf 1‴ besteht aus einem Fußteil 2‴, einem Unterschenkelteil 12‴ sowie einem Oberschenkelteil 13‴, welche aus einem, vorzugsweise einzigen, über mehreren Maschenreihen flachgestrickten Grundgestrickteil 3‴ hergestellt ist. Zudem weist das Fußteil 2‴ ein bevorzugt angestricktes Zehenteil 10' auf. Am oberen Ende des Oberschenkelstrumpfes 1 ‴ist in diesem Ausführungsbeispiel ein Haftband 33‴ angestrickt, so dass ein sicherer Halt des Beinbekleidungsstücks 1‴, insbesondere am Oberschenkel, gewährleistet wird.

Gemäß diesem Ausführungsbeispiel verläuft die das flachgestrickte Grundgestrickteil 3‴ verbindende Längsnaht 5‴ durch die konkrete Anordnung der ersten Funktionszone 4 in dem Grundgestrickteil 3‴ und durch die Ausbildung des Grundgestrickteils 3‴ selbst, im Mittelfußbereich 24, dem Fesselbereich 22, dem Wadenbereich 23, als auch dem Oberschenkelbereich 40 lateral an der Außenseite 8 des Fußes und des Beins eines Trägers des Beinbekleidungsstücks 1‴. Zudem erstreckt sich die Naht 5‴ in den Zehenteil 10' und schließt die Spitze der vorzugsweise angestrickten Fußspitze. Das flachgestrickte Grundgestrickteil 3‴ wird somit durch eine einzige und insbesondere durchgängige Nahtverbindung zu einem geschlossenen Schlauchgestrick zusammengenäht, was deutliche Zeit- und somit Kostenersparnisse mit sich bringt. Mehrere manuell herzustellende Nahtverbindungen sind nicht mehr notwendig. Neben dem deutlich verbesserten Tragekomfort, insbesondere dadurch, dass die Fußsohle 6 sowie die Kniekehle 34 durch die seitliche Anordnung der Naht 5‴ frei von einer störenden Nahtverbindung sind, bringt das gezeigte Ausführungsbeispiel somit den Vorteil einer zeit- und kostensparenden Herstellungsmöglichkeit mit sich.

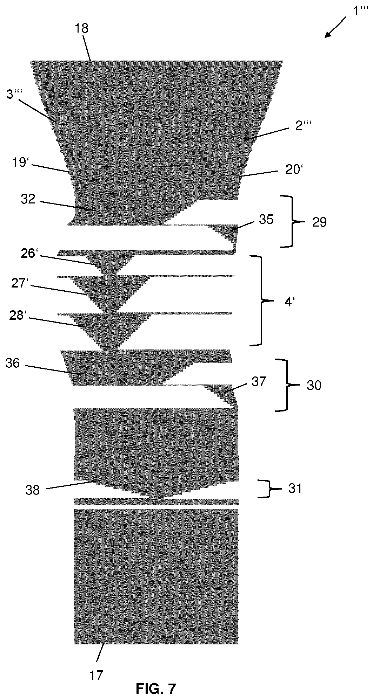

Besonders bevorzugt ist gemäß diesem Ausführungsbeispiel das Grundgestrickteil 3‴ derart ausgebildet, dass die an der Seite des Oberschenkelstrumpfes 1 ‴verlaufende Naht 5‴ insbesondere eine empfindlichen Knöchelzone 16 eines Trägers des Beinbekleidungsstücks 1‴umläuft, diese also nicht kreuzt. Dies geschieht dadurch, dass die zwischen einem Gestrickanfang und einem Gestrickende verlaufenden und gegenüberliegenden Gestrickkanten des flachgestrickten Grundgestrickteils 3‴ asymmetrisch zueinander ausgebildet sind, was später in Figur 7 noch näher erläutert wird.

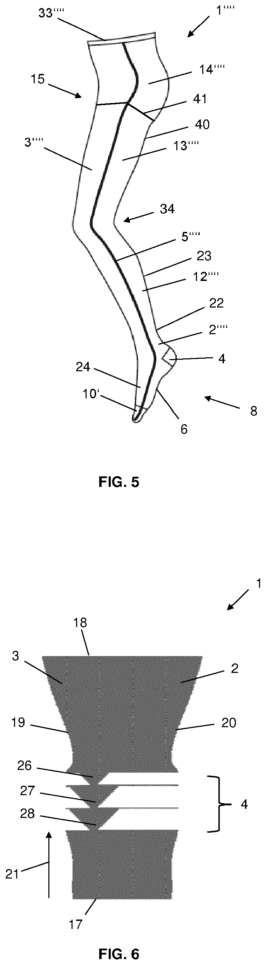

Figur 5 zeigt schließlich ein Beinbekleidungsstück 1‴′in Form einer Strumpfhose mit einem oberen Bund 33"". Die Strumpfhose 1ʺʺ besteht aus einem flachgestrickten Grundgestrickteil 3"", welches ein Fußteil 2"", das wiederum eine erste Funktionszone 4' in Form einer Ferse und ein Zehenteil 10' aufweist, ein Unterschenkelteil 12"", ein Oberschenkelteil 13ʺʺ sowie ein Leibteil 14"" ausbildet. Das flachgestrickte Grundgestrickteil 3ʺʺ ist mittels einer Längsnaht 5ʺʺ zu einem Schlauchgestrick zusammengenäht. Die das flachgestrickte Grundgestrickteil 3‴ verbindende Längsnaht 5‴ verläuft hierbei in einem Mittelfußbereich 24, einem Fesselbereich 22, einem Wadenbereich 23 und einem Oberschenkelbereich 40 lateral an der Außenseite 8 des Fußes und des Beins eines Trägers des Beinbekleidungsstücks 1‴′. Auch im Leibteil 14‴, welches vorzugsweise mittels einer Quernaht 41 an das Oberschenkelteil 13ʺʺ angenäht ist, ist die Naht 5ʺʺ an der Außenseite 15 des Beckens angeordnet. Weder im Bereich der Fußsohle 6, der Kniekehle 34 noch in einem Gesäßbereich, weist das vorzugsweise kompressive und flachgestrickte Beinbekleidungsstücks 1ʺʺ somit eine für den Träger des Beinbekleidungsstücks 3ʺʺ störende und den Tragekomfort verschlechternde Nahtverbindung auf.

Figur 6 zeigt nun in einer schematischen Darstellung den Strickablauf des flachgestrickten Grundgestrickteils 3, wie es zur Bildung des Beinbekleidungsstücks 1, wie es in Figur 1 zu sehen ist, verwendet wird. Das flachgestrickte Grundgestrickteil 3, insbesondere das Fußteil 2, wird mittels zumindest eines Grundstrickfadens, vorzugsweise mittels mehrerer Grundstrickfäden, der bzw. die maschenbildend über mehrere Strickreihen von links nach rechts und/ oder von rechts nach links hinweg in Gestricklängsrichtung 21 verstrickt werden, gebildet. Das auf einer Flachstrickmaschine gestrickte Grundgestrickteil 3 bildet somit ein Flachgestrick aus, welches einen untern Gestrickanfang 17, ein oberes Gestrickende 18 sowie jeweils links und rechts eine Gestrickkante 19 und 20 aufweist. In die Grundstrickmaschen des Grundgestrickteils 3, vorzugsweise in jeder oder jeder n-ten Maschenreihe, ist bevorzugt zumindest ein elastischer Schussfaden eingelegt, um dem Grundgestrickteil 3 kompressive Eigenschaften zu verleihen.

Damit die das Grundgestrickteil 3, insbesondere die Gestrickkanten 19 und 20, verbindende Längsnaht im getragenen Zustand des Beinbekleidungsstücks 1 nun zumindest in einem Fersenbereich außerhalb der Fußsohle angeordnet ist, ist die erste Funktionszone 4, welche zur Aufnahme einer Ferse eines Fußes ausgebildet ist, im Wesentlichen, also vorzugsweise über deren gesamte Breite, zwischen den Gestrickkanten 19 und 20 angeordnet. Die Funktionszone 4, zumindest jener Bereich der Zone 4, welcher im getragenen Zustand im unteren Fersenbereich - also jenem Bereich der Ferse, welcher der Fußsohle zugeordnet ist - angeordnet ist, wird somit nicht von den Gestrickkanten 19 oder 20 geteilt. Dies hätte zur Folge, dass die Naht durch die Funktionszone 4, insbesondere durch den Fersenbereich, welcher der Fußsohle zugeordnet ist, verläuft, was eben erfindungsgemäß vermieden werden soll. Gemäß dem gezeigten Ausführungsbeispiel ist nun die erste Funktionszone 4 zur Aufnahme der Ferse durch mehrere, vorzugsweise drei, Spickel 26, 27 und 28 gebildet. Ein Spickel ist ein im Wesentlichen trapezförmiger Gestrickabschnitt, der sich über mehrere Maschenreihen erstreckt. Hierbei nimmt die Zahl der gebildeten Maschen Reihe für Reihe ab oder zu. Es entstehen somit Teilmaschenreihen mit unterschiedlicher Länge. Dies, insbesondere durch die Ausbildung mehrerer aufeinanderfolgender Spickel, führt schließlich dazu, dass sich ein entsprechender geometrisch definierter Abschnitt ergibt.

Wie in Figur 6 zu sehen ist, sind die Spickel 26, 27 und 28 zur Gänze, also über deren gesamten Breite hinweg, zwischen den Gestrickkanten 19 und 20 angeordnet. D.h. die mehreren Spickel werden nicht durch die Gestrickkanten 19 und 20 geteilt. Bezogen auf eine Maschenreihe im Bereich der Spickel befindet sich somit nur eine einzige Teilmaschenreihe, also keine zweite von der ersten getrennten und beabstandeten Teilreihe, innerhalb einer selben Maschenreihe. Spickelanfang und Spickelende liegen somit zwischen den Gestrickkanten 19 und 20. Abhängig davon, wo die Naht im getragenen Zustand des Grundgestrickteils 3 nun am Fuß des Trägers zum Liegen kommen soll, werden die Spickel 26, 27 und 28 unterschiedlich beabstandet zu den Gestrickkanten 19 und 20 in das Grundgestrickteil 3 eingestrickt. Im gezeigten Beispiel sind die Spickel 26, 27 und 28 deutlich näher zu der linken Gestrickkante 19 angeordnet, was dazu führt, dass die Naht des Beinbekleidungsstücks 1 im getragenen Zustand an der Fußseite eines Trägers zum Liegen kommt. Werden die Spickel beispielsweise mittig im Grundgestrickteil 3 eingestrickt, so kommt die Naht an der Fußoberseite zum Liegen. Die Naht ist jedoch jedenfalls außerhalb der Fußsohle eines Trägers angeordnet.

In Figur 7 ist nun eine schematische Darstellung des Strickablaufs es flachgestrickten Grundgestrickteils des Beinbekleidungsstücks 1", insbesondere ein Ausschnitt daraus, nämlich das Fußteil 2‴, wie es in Figur 4 zu sehen ist, gezeigt. Das flachgestrickte Grundgestrickteil 3‴ mit dem unteren Gestrickanfang 17, dem oberen Gestrickende 18 sowie der linken und rechten Gestrickkante 19' und 20', weist gemäß diesem Ausführungsbeispiel mehrere eingearbeitete Funktionen 4', 29, 30, 31 auf.

Die erste Funktionszone 4' bildet, wie in Figur 6 erläutert, eine Ferse aus und ist auch hier durch drei in das Grundgestrickteil 3‴ eingearbeitete Spickel 26', 27', 28' gebildet. Vor der ersten Funktionszone 4' ist in das Grundgestrickteil 3‴ eine zweite Funktionszone 31 eingestrickt, gebildet durch einen weiteren Spickel 38. Auch dieser Spickel 38 ist über dessen gesamte Breite bevorzugt zwischen den beiden Gestrickkanten 19' und 20' in das Grundgestrickteil 3‴ eingestrickt. Dieser dient dazu einen schrägen Vorfußbereich auszubilden. Letzterer wird durch den mittig im Gestrick angeordneten Spickel 38 und der dazu versetzt im Grundgestrickteil 3‴ angeordnete Spickel 26', 27' und 28' der Ferse gebildet. Des Weiteren ist unmittelbar vor und nach der ersten Funktionszone 4' eine dritte und vierte Funktionszone 29, 30 eingestrickt. Auch diese Zonen sind durch Spickel 32 und 35 sowie 36 und 37 gebildet. Diese beiden Zonen dienen dazu die Anzahl an Maschen im Mittelfußbereich, insbesondere im Bereich des Spanns, zur reduzieren, so dass es dort im getragenen Zustand des Beinbekleidungsstücks zu einer geringeren bis zu keiner Faltenbildung durch das Grundgestrickteil 3‴ kommt.

Die Erfindung ist nicht auf die beschriebenen Ausführungsbeispiele beschränkt, sondern umfasst alle Ausführungen gemäß dem Gegenstand des unabhängigen Anspruchs Des Weiteren sind alle Merkmale aller beschriebenen und dargestellten Ausführungsbeispiele miteinander kombinierbar.

## Patentansprüche

1. Beinbekleidungsstück (1, 1', 1", 1‴, 1""), aufweisend ein zumindest ein Fußteil (2, 2', 2", 2"',2"") ausbildendes flachgestricktes Grundgestrickteil (3, 3', 3", 3‴, 3""), mit zumindest einer ersten in das Grundgestrickteil (3, 3', 3", 3‴, 3"") eingestrickten Funktionszone (4, 4'), welche zur Aufnahme einer Ferse eines Fußes ausgebildet ist, wobei das flachgestrickte Grundgestrickteil (3, 3', 3", 3‴, 3"") mittels einer Längsnaht (5, 5', 5", 5‴, 5"") zu einem Schlauchgestrick zusammengenäht ist, **dadurch gekennzeichnet, dass** die zumindest eine erste Funktionszone (4, 4') derart in das Grundgestrickteil (3, 3', 3", 3‴, 3"") eingestrickt ist, dass die das Grundgestrickteil (3, 3', 3", 3‴, 3"") verbindende Längsnaht (5, 5', 5", 5‴, 5"") im getragenen Zustand des Beinbekleidungsstücks (1, 1', 1", 1‴, 1"") zumindest in einem Fersenbereich außerhalb der Fußsohle (6) angeordnet ist.

2. Beinbekleidungsstück (1, 1', 1", 1‴, 1"") nach Anspruch 1, **dadurch gekennzeichnet, dass** sich die Längsnaht (5, 5', 5", 5‴, 5"") im getragenen Zustand des Beinbekleidungsstücks (1, 1', 1", 1‴, 1"") medial (7) oder lateral (8) an der Seite des Fußes und/ oder über die Fußoberseite (9) erstreckt

3. Beinbekleidungsstück (1', 1", 1‴, 1"") nach Anspruch 1 oder 2, **dadurch gekennzeichnet, dass** das flachgestrickte Grundgestrickteil (3', 3", 3‴, 3"") zusätzlich ein Zehenteil (10, 10') ausbildet, wobei das Zehenteil (10, 10') mittels einer Quernaht (11) zu einer geschlossenen Zehenkappe zusammengenäht ist.

4. Beinbekleidungsstück (1‴, 1"") nach Anspruch 3, **dadurch gekennzeichnet, dass** die Längsnaht (5‴, 5"") des Fußteils (2", 2‴) und die Quernaht (11) des Zehenteils (10') als eine durchgängige Naht ausgebildet sind.

5. Beinbekleidungsstück (1', 1", 1‴, 1"") nach einem der vorherigen Ansprüche, **dadurch gekennzeichnet, dass** das flachgestrickte Grundgestrickteil (3', 3", 3‴, 3"") zusätzlich ein Unterschenkelteil (12', 12",12‴, 12"") ausbildet, wobei sich die Längsnaht (5', 5", 5‴, 5"") im getragenen Zustand des Beinbekleidungsstücks (1', 1", 1‴, 1"") medial (7) oder lateral (8) an der Seite des Unterschenkels und/ oder sich über die Vorderseite (25) erstreckt.

6. Beinbekleidungsstück (1‴, 1"") nach Anspruch 5, **dadurch gekennzeichnet, dass** das flachgestrickte Grundgestrickteil (3‴, 3"") zusätzlich ein Oberschenkelteil (13‴, 13"") ausbildet, wobei sich die Längsnaht (5‴, 5"") im getragenen Zustand des Beinbekleidungsstücks (1‴, 1"") medial (7) oder lateral (8) an der Seite des Oberschenkels und/ oder sich über die Vorderseite (25) des Oberschenkels erstreckt.

7. Beinbekleidungsstück (1"") nach Anspruch 6, **dadurch gekennzeichnet, dass** das flachgestrickte Grundgestrickteil (3"") zusätzlich ein Leibteil (14"") ausbildet, wobei sich die Längsnaht (5"") im getragenen Zustand des Beinbekleidungsstücks (1"") an der Seite (15) des Beckens und/ oder sich über die Vorderseite (25) des Beckens erstreckt.

8. Beinbekleidungsstück (1‴) nach einem der vorherigen Ansprüche, **dadurch gekennzeichnet, dass** das das flachgestrickte Grundgestrickteil (3‴) zumindest eine zweite in das Grundgestrickteil (3‴) eingestrickten Funktionszone (29, 30, 31) aufweist, welche eine höhere Elastizität in Längs- und/ oder Querrichtung aufweist.

9. Beinbekleidungsstück (1, 1', 1", 1"") nach einem der vorherigen Ansprüche, **dadurch gekennzeichnet, dass** die zwischen einem Gestrickanfang (17) und einem Gestrickende (18) verlaufenden und gegenüberliegenden Gestrickkanten (19, 20) des flachgestrickten Grundgestrickteils (3, 3', 3", 3"") symmetrisch zueinander ausgebildet sind.

10. Beinbekleidungsstück (1‴) nach einem der Ansprüche 1 bis 9, **dadurch gekennzeichnet, dass** die zwischen einem Gestrickanfang (17) und einem Gestrickende (18) verlaufenden und gegenüberliegenden Gestrickkanten (19', 20') des flachgestrickten Grundgestrickteils (3‴) asymmetrisch zueinander ausgebildet sind.

11. Beinbekleidungsstück (1, 1', 1", 1‴, 1"") nach einem der vorherigen Ansprüchen, **dadurch gekennzeichnet, dass** das flachgestrickte Grundgestrickteil (3, 3', 3", 3‴, 3"") durch einen oder mehrere maschenbildende Grundstrickfäden sowie zumindest einen eingelegten Schussfaden gebildet ist.

12. Beinbekleidungsstück (1, 1', 1", 1‴, 1"") nach einem der vorherigen Ansprüchen, **dadurch gekennzeichnet, dass** das flachgestrickte Grundgestrickteil (3, 3', 3", 3‴, 3"") in Gestricklängsrichtung (21) des Beinbekleidungsstücks (1, 1', 1", 1‴, 1"") einen graduellen Kompressionsverlauf aufweist.

13. Beinbekleidungsstück (1, 1', 1", 1‴, 1"") nach Anspruch 12, **dadurch gekennzeichnet, dass** die durch das flachgestrickte Grundgestrickteil (3, 3', 3", 3‴, 3"") erzeugten kompressiven Drücke in einem Fesselbereich (22) des Beinbekleidungsstücks zwischen 10 und 60 mmHg und in einem Wadenbereich (23) zwischen 5 und 50 mmHg betragen.

14. Beinbekleidungsstück (1, 1', 1", 1‴, 1"") nach einem der vorherigen Ansprüche, **dadurch gekennzeichnet, dass** das das Beinbekleidungsstück (1, 1', 1", 1‴, 1"") einteilig oder mehrteilig ausgebildet ist.

15. Beinbekleidungsstück (1, 1', 1", 1‴, 1"") nach einem der vorherigen Ansprüche, **dadurch gekennzeichnet, dass** das Beinbekleidungsstück (1, 1', 1", 1‴, 1"") ein Füßling, eine Socke, ein Strumpf (1, 1', 1", 1‴)eine Strumpfhose (1""), eine Fußbandage, oder ein Strickteil einer Fußorthese ist.

## Claims

1. A leg garment (1, 1', 1", 1‴, 1ʺʺ) comprising a flat-knitted basic knitted fabric part (3, 3', 3'', 3‴, 3ʺʺ) that forms at least a foot part (2, 2', 2", 2‴, 2ʺʺ), with at least one first functional zone (4, 4') that is knitted into the basic knitted fabric part (3, 3', 3", 3‴, 3ʺʺ) and designed for accommodating a heel of a foot, wherein the flat-knitted basic knitted fabric part (3, 3', 3", 3‴, 3ʺʺ) is sewn together by means of a longitudinal seam (5, 5', 5'', 5''', 5ʺʺ) so as to form a tubular knitted fabric, **characterized in that** the at least one first functional zone (4, 4') is knitted into the basic knitted fabric part (3, 3', 3'', 3‴, 3ʺʺ) in such a way that, in the worn state of the leg garment ( 1, 1', 1" , 1‴, 1''''), the longitudinal seam (5, 5', 5", 5‴, 5ʺʺ) connecting the basic knitted fabric part (3, 3', 3", 3‴, 3ʺʺ) is arranged outside the sole (6) of the foot at least in a heel region.

2. The leg garment (1, 1', 1", 1‴, 1ʺʺ) according to claim 1, **characterized in that** the longitudinal seam (5, 5', 5'', 5''', 5ʺʺ) extends medially (7) or laterally (8) on the side of the foot and/or over the upper side (9) of the foot in the worn state of the leg garment (1, 1', 1'', 1''', 1ʺʺ).

3. The leg garment (1', 1", 1‴, 1ʺʺ) according to claim 1 or 2, **characterized in that** the flat-knitted basic knitted fabric part (3', 3ʺ, 3‴, 3ʺʺ) additionally forms a toe part (10, 10'), wherein the toe part (10, 10') is sewn together by means of a transverse seam (11) so as to form a closed toe cap.

4. The leg garment (1‴, 1ʺʺ) according to claim 3, **characterized in that** the longitudinal seam (5‴, 5'''') of the foot part (2'', 2‴) and the transverse seam (11) of the toe part (10') are realized in the form of a continuous seam.

5. The leg garment (1', 1", 1‴, 1ʺʺ) according to one of the preceding claims, **characterized in that** the flat-knitted basic knitted fabric part (3', 3ʺ, 3‴, 3ʺʺ) additionally forms a lower leg part (12', 12'', 12‴, 12ʺʺ), wherein the longitudinal seam (5', 5", 5‴, 5'''') extends medially (7) or laterally (8) on the side of the lower leg and/or over the front side (25) in the worn state of the leg garment (1', 1", 1‴, 1ʺʺ).

6. The leg garment (1‴, 1ʺʺ) according to claim 5, **characterized in that** the flat-knitted basic knitted fabric part (3‴, 3ʺʺ) additionally forms a thigh part (13‴, 13ʺʺ), wherein the longitudinal seam (5‴, 5ʺʺ) extends medially (7) or laterally (8) on the side of the thigh and/or over the front side (25) of the thigh in the worn state of the leg garment 1‴, 1ʺʺ).

7. The leg garment (1ʺʺ) according to claim 6, **characterized in that** the flat-knitted basic knitted fabric part (3ʺʺ) additionally forms an abdominal part (14ʺʺ), wherein the longitudinal seam (5ʺʺ) extends on the side (15) of the pelvis and/or over the front side (25) of the pelvis in the worn state of the leg garment (1ʺʺ).

8. The leg garment (1‴) according to one of the preceding claims, **characterized in that** the flat-knitted basic knitted fabric part (3‴) has at least one second functional zone (29, 30, 31), which is knitted into the basic knitted fabric part (3‴) and has a higher elasticity in the longitudinal and/or transverse direction.

9. The leg garment (1, 1', 1'', 1ʺʺ) according to one of the preceding claims, **characterized in that** the knitted fabric edges (19, 20) of the flat-knitted basic knitted fabric part (3, 3', 3'', 3ʺʺ), which extend between a knitted fabric beginning (17) and a knitted fabric end (18) and lie opposite of one another, are formed symmetrically to one another.

10. The leg garment (1‴) according to one of claims 1 to 9, **characterized in that** the knitted fabric edges (19', 20') of the flat-knitted basic knitted fabric part (3‴), which extend between a knitted fabric beginning (17) and a knitted fabric end (18) and lie opposite of one another, are formed asymmetrically to one another.

11. The leg garment (1, 1', 1", 1‴, 1'''') according to one of the preceding claims, **characterized in that** the flat-knitted basic knitted fabric part (3, 3', 3'', 3‴, 3ʺʺ) is formed by one or more stitch-forming basic knitting threads and at least one inserted weft thread.

12. The leg garment (1, 1', 1", 1‴, 1'''') according to one of the preceding claims, **characterized in that** the flat-knitted basic knitted fabric part (3, 3', 3'', 3‴, 3ʺʺ) has a gradual compression curve in the longitudinal direction (21) of the knitted fabric of the leg garment (1, 1', 1ʺ, 1‴, 1‴′).

13. The leg garment (1, 1', 1ʺ, 1‴, 1'''') according to claim 12, **characterized in that** the compressive pressures generated by the flat-knitted basic knitted fabric part (3, 3', 3", 3‴, 3ʺʺ) lie between 10 and 60 mmHg in an ankle region (22) of the leg garment and between 5 and 50 mmHg in a calf region (23).

14. The leg garment (1, 1', 1", 1‴, 1'''') according to one of the preceding claims, **characterized in that** the leg garment (1, 1', 1", 1''', 1ʺʺ) is formed in one piece or in multiple pieces.

15. The leg garment (1, 1', 1", 1‴, 1'''') according to one of the preceding claims, **characterized in that** the leg garment (1, 1', 1", 1‴, 1ʺʺ) is a sock liner, a sock, a stocking (1, 1', 1", 1'''), a pair of tights (1ʺʺ), a foot bandage or a knitted part of a foot orthosis.

## Revendications

1. Pièce d'habillage de jambe (1, 1', 1", 1‴, 1ʺʺ), comportant une partie en tricot de base (3, 3', 3", 3‴, 3ʺʺ) tricotée à plat, constituant au moins une partie pied (2, 2̋, 2‴,2‴), pourvue d'au moins une première zone fonctionnelle (4, 4') emmaillée dans la partie en tricot de base (3, 3', 3", 3‴, 3ʺʺ), laquelle est conçue pour recevoir un talon d'un pied, la partie en tricot de base (3, 3', 3", 3‴, 3ʺʺ) tricotée à plat étant cousue au moyen d'une couture longitudinale (5, 5', 5", 5‴, 5ʺʺ) en un tricot tubulaire, **caractérisée en ce que** l'au moins une première zone fonctionnelle (4, 4') est emmaillée dans la partie en tricot de base (3, 3', 3", 3‴, 3ʺʺ) de telle sorte que, lorsque la pièce d'habillage de jambes (1, 1', 1'', 1‴, 1ʺʺ) est portée, la couture longitudinale (5, 5', 5", 5‴, 5ʺʺ) reliant la partie en tricot de base (3, 3', 3", 3‴, 3ʺʺ) soit placée au moins dans une zone du talon à l'extérieur de la plante du pied (6).

2. Pièce d'habillage de jambe (1, 1', 1", 1‴, 1ʺʺ) selon la revendication 1, **caractérisée en ce que** lorsque la pièce d'habillage de jambes (1, 1', 1", 1‴, 1ʺʺ) est portée, la couture longitudinale (5, 5', 5", 5‴, 5ʺʺ) s'étend dans la direction médiale (7) ou latérale (8) sur le côté du pied et / ou par-dessus le dessus du pied (9).

3. Pièce d'habillage de jambe (1, 1', 1", 1‴, 1ʺʺ) selon la revendication 1 ou 2, **caractérisée en ce que** la partie en tricot de base (3', 3", 3‴, 3ʺʺ) tricotée à plat constitue additionnellement une partie orteil (10, 10'), la partie orteil (10, 10') étant cousue au moyen d'une couture transversale (11) en un embout protège-orteil fermé.

4. Pièce d'habillage de jambe ( 1‴, 1‴′) selon la revendication 3, **caractérisée en ce que** la couture longitudinale (5 ‴, 5'''') de la partie pied (2", 2‴) et la couture transversale (11) de la partie orteil (10') est conçue sous la forme d'une couture continue.

5. Pièce d'habillage de jambe (1, 1', 1", 1‴, 1ʺʺ) selon l'une quelconque des revendications précédentes, **caractérisée en ce que** la partie en tricot de base (3, 3', 3", 3‴, 3ʺʺ) tricotée à plat constitue additionnellement une partie jambe inférieure (12', 12",12‴, 12ʺʺ), lorsque la pièce d'habillage de jambes (1, 1', 1'', 1‴, 1ʺʺ) est portée, la couture longitudinale (5, 5', 5", 5‴, 5ʺʺ) s'étendant en direction médiale (7) ou latérale (8) sur le côté de la jambe inférieure et / ou s'étendant par-dessus la face avant (25).

6. Pièce d'habillage de jambe ( 1‴, 1ʺʺ) selon la revendication 5, **caractérisée en ce que** la partie en tricot de base (3‴, 3‴′) tricotée à plat constitue additionnellement une partie cuisse (13‴, 13ʺʺ), lorsque la pièce d'habillage de jambes ( 1‴, 1ʺʺ) est portée, la couture longitudinale (5‴, 5ʺʺ) s'étendant en direction médiale (7) ou latérale (8) sur le côté de la cuisse et / ou s'étendant par-dessus la face avant (25) de la cuisse.

7. Pièce d'habillage de jambe (1ʺʺ) selon la revendication 6, **caractérisée en ce que** la partie en tricot de base (3'''') tricotée à plat constitue additionnellement une partie corps (14ʺʺ),lorsque la pièce d'habillage de jambes (1ʺʺ) est portée, la couture longitudinale (5ʺʺ) s'étendant sur le côté (15) du bassin et / ou s'étendant par-dessus la face avant (25) du bassin.

8. Pièce d'habillage de jambe (1‴) selon l'une quelconque des revendications précédentes, **caractérisée en ce que** la partie en tricot de base (3‴) tricotée à plat comporte au moins une deuxième zone fonctionnelle (29, 30, 31) emmaillée dans la partie en tricot de base (3‴), laquelle fait preuve d'une élasticité supérieure dans la direction longitudinale et / ou transversale.

9. Pièce d'habillage de jambe (1, 1', 1", 1ʺʺ) selon l'une quelconque des revendications précédentes, **caractérisée en ce que** les arêtes de tricot (19, 20) de la partie en tricot de base (3, 3', 3", 3ʺʺ) tricotée à plat, opposées et s'écoulant entre un début de tricot (17) et une fin de tricot (18) sont conçues en étant symétriques l'une par rapport à l'autre.

10. Pièce d'habillage de jambe (1‴) selon l'une quelconque des revendications 1 à 9, **caractérisée en ce que** les arêtes de tricot (19', 20') de la partie en tricot de base (3‴) tricotée à plat s'écoulant entre un début de tricot (17) et une fin de tricot (18) et opposées sont conçues en étant asymétriques l'une par rapport à l'autre.

11. Pièce d'habillage de jambe (1, 1', 1", 1‴, 1ʺʺ) selon l'une quelconque des revendications précédentes, **caractérisée en ce que** la partie en tricot de base (3, 3', 3", 3‴, 3ʺʺ) tricotée à plat est constituée d'un ou de plusieurs fils de tricot de base formant des mailles ainsi que d'au moins un fil de trame inséré.

12. Pièce d'habillage de jambe (1, 1', 1", 1‴, 1ʺʺ) selon l'une quelconque des revendications précédentes, **caractérisée en ce que** dans la direction longitudinale de tricot (21) de la pièce d'habillage de jambes (1, 1', 1", 1‴, 1ʺʺ), la partie en tricot de base (3, 3', 3‴, 3‴, 3ʺʺ) tricotée à plat présente un trajet de compression graduel.

13. Pièce d'habillage de jambe (1, 1', 1", 1‴, 1ʺʺ) selon la revendication 12, **caractérisée en ce que** les pressions compressives exercées par la partie en tricot de base (3, 3', 3‴, 3‴, 3ʺʺ) tricotée à plat dans une zone de cheville (22) de la pièce d'habillage de jambes se situent entre 10 et 60 mmHg et dans une zone du mollet (23) se situent entre 5 et 50 mmHg.

14. Pièce d'habillage de jambe (1, 1', 1", 1‴, 1ʺʺ) selon l'une quelconque des revendications précédentes, **caractérisée en ce que** la pièce d'habillage des jambes (1, 1', 1", 1‴, 1ʺʺ) est conçue en une partie ou en plusieurs parties.

15. Pièce d'habillage de jambe (1, 1', 1", 1‴, 1ʺʺ) selon l'une quelconque des revendications précédentes, **caractérisée en ce que** la pièce d'habillage de jambe (1, 1', 1", 1‴, 1ʺʺ) est un chausson, une socquette, une chaussette (1, 1', 1", 1‴), un collant (1ʺʺ), un bandage de pied, ou une partie tricotée d'une orthèse de pied.
